(19) Europäisches Patentamt
European Patent Office
Office européen des brevets

(11) **EP 3 876 869 B1**

(12) **EUROPEAN PATENT SPECIFICATION**

(45) Date of publication and mention
of the grant of the patent:
**25.09.2024  Bulletin 2024/39**

(21) Application number: **19829703.8**

(22) Date of filing: **05.11.2019**

(51) International Patent Classification (IPC):
***A61F 2/24*** *(2006.01)*

(52) Cooperative Patent Classification (CPC):
**A61F 2/2415**

(86) International application number:
**PCT/PL2019/000100**

(87) International publication number:
**WO 2020/096469 (14.05.2020 Gazette 2020/20)**

(54) **THE SYSTEM OF AN ELEMENT USED FOR THE CREATION OF HEART VALVE, THE METHOD OF MANUFACTURING OF MODIFIED BACTERIAL CELLULOSE (BC), THE SET AND THE ELEMENT USED IN CARDIO SURGERY**

SYSTEM EINES ELEMENTS ZUR HERSTELLUNG VON HERZKLAPPEN, VERFAHREN ZUR HERSTELLUNG VON MODIFIZIERTER BAKTERIENZELLULOSE (BC), SATZ UND ELEMENT ZUR VERWENDUNG IN DER HERZCHIRURGIE

SYSTÈME D'UN ÉLÉMENT UTILISÉ POUR LA CRÉATION D'UNE VALVULE CARDIAQUE, PROCÉDÉ DE FABRICATION DE CELLULOSE BACTÉRIENNE MODIFIÉE (BC), ENSEMBLE ET ÉLÉMENT UTILISÉS EN CHIRURGIE CARDIO-VASCULAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.11.2018  PL 42765218**

(43) Date of publication of application:
**15.09.2021  Bulletin 2021/37**

(73) Proprietors:
• **Gdanski Uniwersytet Medyczny**
**80-210 Gdansk (PL)**
• **Bowil Biotech Sp. z o.o.**
**84-120 Wladyslawowo (PL)**
• **Centrum Techniki Okretowej S.A.**
**80-392 Gdansk (PL)**
• **Politechnika Gdanska**
**80-233 Gdansk (PL)**
• **Uniwersytet Gdanski**
**80-309 Gdansk (PL)**
• **Fundacja Rozwoju Kardiochirurgii**
**Im. Prof. Zbigniewa Religi**
**41-800 Zabrze (PL)**

(72) Inventors:
• **SIONDALSKI, Piotr**
**83-050 Pregowo (PL)**
• **KOLACZKOWSKA, Magdalena**
**81-157 Gdynia (PL)**
• **WILANDT, Waldemar**
**84-141 Jurata (PL)**
• **BOBINSKI, Dariusz**
**84-141 Jurata (PL)**
• **DLUGA, Aldona**
**84-140 Jastarnia (PL)**
• **DAWIDOWSKA, Kinga**
**77-100 Bytów (PL)**
• **DITRICH, Michal**
**80-298 Gdansk (PL)**
• **WILCZYNSKI, Leszek**
**80-419 Gdansk (PL)**
• **STAROSZCZYK, Hanna**
**30-049 Kraków (PL)**
• **DEDERKO-KANTOWICZ, Paulina**
**76-009 Bonin (PL)**
• **MALINOWSKA-PANCZYK, Edyta**
**81-109 Gdynia (PL)**
• **SOMMER, Agata**
**11-440 Reszel (PL)**
• **SINKIEWICZ, Izabela**
**83-000 Pruszcz Gdanski (PL)**

- **KOLODZIEJSKA, Ilona**
  **81-661 Gdynia (PL)**
- **SZKODO, Marek**
  **80-172 Gdansk (PL)**
- **STANISLAWSKA, Alicja**
  **80-277 Gdansk (PL)**
- **BORMAN, Andrzej**
  **80-275 Gdansk (PL)**
- **SWIERGIEL, Artur Hugo**
  **02-002 Warszawa (PL)**
- **PALCZYNSKA, Paulina**
  **80-807 Gdansk (PL)**
- **JABLONSKI, Grzegorz**
  **10-455 Olsztyn (PL)**
- **GLAC, Wojciech**
  **84-200 Wejherowo (PL)**

- **WILCZEK, Piotr**
  **43-600 Jaworzno (PL)**
- **GAWLIKOWSKI, Maciej**
  **41-800 Zabrze (PL)**

(74) Representative: **JWP Patent & Trademark Attorneys**
**Sienna Center, ul. Zelazna 28/30**
**00-833 Warszawa (PL)**

(56) References cited:
**WO-A1-2013/119912    US-B1- 6 328 763**

## Description

The object of the invention is an element for manufacturing a heart valve, a method for producing modified bacterial cellulose (BC) film by using a *Gluconacetobacter xylinus* strain for manufacturing the element and a kit comprising the element for use as a heart valve in cardio surgery, as defined by claims 1, 5 and 9 respectively.

Prostheses of heart valves may be manufactured from artificial materials - then they are known as mechanical prostheses, and from biological materials - then they are known as biological prostheses.

For patients with valvular defects in whom all methods of conservative treatment were applied, the only way of therapy is the implantation of heart valve prosthesis.

Problems related to the implantation of these protheses are, above all, degeneration of biological prosthesis and clotting on mechanical prosthesis. The valve built of BC is a solution for these problems, since, which was proved by the research conducted, no clotting takes place on its surface and this material is characterised by high durability in comparison to natural tissues.

Prostheses currently available on the market are very expensive, so, as a result, the choice is not only dependent on the biological qualities but also on the costs related to the implantation of various prostheses. The solution proposed by us provides the doctor and the patient with the possibility of application of a cheap and safe treatment.

The currently known and used valves possess multiple faults. They require an additional treatment, i.e., anti-clotting treatment, they are subject to degeneration and, therefore, a new material is being searched for.

The descriptions of inventions present *inter alia* an elastic stent of biologically joined heart valves which allows a concurrent implantation of biological prosthesis of aortic valve and mitral valve - PL 180925 B. EP 1083845 B discloses an upgraded tricuspid mechanical valve, in which the casing of the valve has an articulated mechanism, what allows the spinning of cusps and their supporting. Another invention description PL 229562 B discloses a mechanical tricuspid heat valve.

The utility of BC in the healing of various wounds was already proven in the 1990s. As regards wound dressing and superficial/surface application, BC is a non-toxic, non-irritating, hypoallergic, non-pyrogenic and biocompatible material.

BC may become the material used for manufacturing of durable, inner implants (vascular prostheses and heart valve prostheses), used especially in vascular surgery and cardio surgery.

Bacterial cellulose is a non-toxic, non-irritating, hypoallergic, non-pyrogenic and biocompatible material. It is characterised by a high mechanical strength.

There are many attempts to substitute the currently used cardio-vascular implants, made from zoonotic and artificial materials, with BC membrane prostheses.

A great value of BC is its impermeability even for the slightest blood cellular elements (the diameter of thrombocytes reaches the values of 1000 - 2000 nm and the size of the bio cellulose fibre net reaches from 20 to 100 nm). The surface of BC is therefore completely smooth for blood, which reduces the possibility of clotting. This attribute should also make the outgrow of BC through the own cells of the host organism impossible.

To date, the inventions concerning BC *in situ* modification methods were described, and this modification method is based on the application of chemical additives to the culture medium. In biomedicine, these inventions are applied most frequently as new generation dressing materials especially in hard to heal wounds and as a material substituting natural tissues, *inter alia* cartilage tissue.

The description of patent PL 190961 B1 presents a method for obtaining a modified BC membrane, by adding a culture medium of poly-amino-saccharides, especially chitosan, in a microcrystalline form, of an average molecular weight of 20500 kDa and deacetylation degree of 70-95%, in a quantity of 0,1-30 parts by weight. The BC membranes obtained according to the invention possess glucosamines in the chain, formed as a result of the degradation of poly-amino-saccharides or their derived substances. The composite material derived in this manner is characterised by bioactivity and biocompatibility desired for biomedical application and it is biodegradable.

The description of patent application EP 346507 A discloses a method for manufacturing a modified BC, through the application of carboxymethylcellulose (CMC) as an additive to the culture medium, used in the quantity of 0.1% - 5% w/v. The BC membranes created in the presence of CMC are characterised by higher dry and wet weight, as well as increased water absorbency in comparison to BC membranes synthetized without the addition of CMC.

In patent PL 214844 B1 a method of modification of BC membranes which consists in enriching a culture medium with 0.25-1% CMC, and then, after the end of cultivation, oxidizing the membrane with 1% solution of sodium periodate at a temperature of 22°C for 2 hours is presented. The membranes obtained are characterised by increased water absorbency and a higher water retention capacity, as a result of which a thicker, better hydrated material is created.

From the description provided in patent application US 2009/0220560 we know a dressing obtained from BC, coated with nano silver, which provides polymer with antibacterial properties. The invention discloses the obtaining of cellulose fibres with the use of *Acetobacter xylinum BPR 2001* strain. After cleaning, the cellulose fibres are treated, in this order, with 0.16 M sodium periodate, 1% amino thiourea in acetic acid, 1% silver protein in 2% sodium borate and silver salt in ammonia water at a temperature of 95°C. The nano silver molecules formed as a result of the series of reactions cover BC fibres.

In the description of patent PL 216702 B1 a method for the manufacturing of biomaterial with cartilage properties with the use of the BC obtained in the process of cultivation of *G. xylinus* bacteria on a stationary medium is disclosed. After obtaining and cleaning, the BC membrane is modelled into a spatial structure of a desired shape and subjected to modification consisting in treating it with 30% aqueous sodium lye solution, rinsing in distilled water, 10% aqueous acetic acid solution and repeated rinsing in distilled water until the cellulose material reaches pH 5.6 - 6.8. The material obtained this way has a very high mechanic strength, corresponding to the natural cartilage tissue, is biocompatible with human body and is non-allergic.

For applications in cardio surgery and vascular surgery, the materials manufactured as described above are unsuitable because they may be characterised by reduced human biocompatibility, inadequate mechanical properties, inadequate morphology and accelerated biodegradability.

In the description of patent application WO2016083352 (PCT/EP2015/077464), a heart valve prosthesis built up on a frame stent with leaflets made of particularly treated BC is presented. The method of preparing cellulosic material described in patent application WO2016083352 consists in producing a molded body made of bacterial cellulose, including the steps of mechanically pressing parts of the parts of molded body at temperatures in the range of 10 ° C to 100 ° C and pressing in the range of 0.0005 to 1.5 MPa by 10-200 min, treatment of the produced material with a solution consisting of: 20 weight % to 50 weight % glycerol and 50 weight % to 80 weight % alcohol / water mixture and drying the treated molded body. This technique is different from the method described in the present application for modifying the cellulose film which consists in initial drying and additional final soaking of the prepared structure in endotoxin-free water. The process of additional soaking of previously dried cellulosic material is important and has a significant impact on its strength parameters.

In the description of patent application WO2013119912 (PCT/US2013/025287), a heart valve prosthesis molded on an expanding frame is presented. The cellulose material used as a valve described in WO2013119912 is a composite system - cellulose material coupled to the frame of a defined structure. In addition, composite materials based on bacterial cellulose with the addition of silicone in a ratio of 60 : 40 (% by weight) have been described. Claim 22 describes a method of forming cellulosic material in a glass container by pouring a cellulose-based mixture.

In the description of patent application US 2009/0222085 A1 a heart valve molded on a stent as well as a particular BC treatment are presented. The description in US Patent application number 2009/0222085 A1 relates to a method of modifying cellulosic material of plant origin in order to obtain a structure suitable for producing a heart valve. It is important whether the cellulose is of plant or bacterial origin because of the chemical and mechanical properties that the particular material possess.

The invention described in US6328763B1 relates generally to a new and improved heart valve tissue pattern and heart valve for semilunar heart valve reconstruction. The physical profile of the valve of the present invention is a characteristic defined in part by the length of the lobes. The unique two-dimensional trefoil tissue pattern of the invention can be made from any appropriate materials such as, for example, autologous tissue including, for example, pericardium, fascia lata, rectus sheath (the fibrous tissue enveloping the abdominal muscles). Additional acceptable materials include heterograft (bovine, porcine, or other animal tissue) pericardium, synthetic materials, bioengineered materials, or any other like or suitable materials having appropriate plasticity and characteristics, as will be appreciated by those skilled in the art.

The method of modification of BC film in order to use it as a material for the production of bio prostheses in the circulatory system, obtained during stationary cultivation with the use of *G. xylinus* strain is characterised, according to the invention, in that sterile BC membranes are dried to a constant mass at room temperature of not more than 25°C, then soaked in sterile distilled water at room temperature for no more than 120 minutes and stored under sterile conditions.

A variation of the invention is the BC film dried to a constant mass at room temperature of no more than 25°C and then exposed to UV-C radiation with a total power of 12W and a maximum emission of 254 nm for a period of no more than 30 minutes. After exposure, the films are soaked in sterile distilled water at room temperature for a period not exceeding 120 minutes and stored at sterile conditions.

Due to the use of the modification method according to the invention, it is possible to obtain a biocompatible material with the properties comparable to the natural tissues building walls of blood vessels or valves, used in cardio surgery and vascular surgery for the production of bioimplants. In contrast to unmodified membranes, the material modified according to the invention is characterized by a water content similar to natural tissues and much higher mechanical strength. The material obtained according to the invention also has increased resistance to degradation processes in the human body.

The heart valve forming element is made of one sheet of biocompatible material, favourably polymer, favourably BC and is a flat, favourably biconnected figure, favourably with three axes of symmetry intersecting at one point, with a centrally made hole with three equal sides, not necessarily straight, forming a figure with the symmetry analogous to that of an equilateral triangle. The axes of the symmetry of the element and the hole coincide. The edges of the hole are the first internal sides of the first areas of the element, and the first areas of the element being essentially quadrilateral in shape. Between the first areas of the element there are, directly adjacent to them, second areas of the element, whose outer edges are favourably rounded convexly. The second sides of the first areas are generally perpendicular to the first sides of the first areas, with the ratio of the length of the first side of the first area to the second side of the first area being between $\pi/3$ and $2\pi/3$. The measure of the angles between the second and third sides of the first areas is $2\pi/3$ to $5\pi/6$. The length of the outer sides of the first areas is at least equal to the length of the first inner sides of the first areas. The ratio of the radius of the circle coinciding with the centre of the symmetry of the hole and tangent to the outer side of the first area at mid-height to the length of the second side of the first area is between $2\pi/9$ and $5\pi/6$.

The object of the invention is to provide a new heart valve and a new method of BC modification as the material which the valve is made of. Moreover, the invention also aims to use the new valve in cardiac surgery.

The object of the invention is an element for manufacturing a heart valve which contains:

- a flat sheet of BC with three axes of symmetry, with a centrally made hole with three equal sides, forming a figure with the symmetry of an equilateral triangle,
- edges of the hole are inner sides of first areas of the element,
- the first areas of the element are quadrilateral,
- second areas of the element are placed between the first areas of the element adjacent thereto,
- the second areas of the element are circle slices having a central angle which is from $2\pi/3$ to $5\pi/6$ and radii equal to lengths of edges of second sides of the first areas of the element,
- the edges of the second sides of the first areas of the element are perpendicular to the inner sides of the first areas of the element,
- length of outer sides of the first areas of the element is equal or bigger as compared to the length of the first inner sides of the first areas of the element,
- a ratio of the length of radius of the circle with its centre coinciding with a centre of symmetry of the central hole and tangent to the outer side of the first areas of the element, at mid-height length, to the length of the second sides of the first areas of the element is between $2\pi/9$ and $5\pi/6$,

 -- a ratio of the length of the first inner sides of the first areas of the element to the second sides of the first areas of the element is between $\pi/3$ and $2\pi/3$.

The element is preferably a bi-coherent figure.
The element where the axes of symmetry of the element and the hole overlap.
The element where the first areas meet the second areas on the outer side of the element and the element has strengthening knobs, which are favourably semi-circular.

The object of the invention is a method for producing the modified BC film defined above by using a *G. xylinus* strain, where:

- sterilized BC film is laid on a flat surface and dried to constant weight at room temperature not exceeding 25°C,
- it is sterilized at 121°C for 20 minutes,
- the resulting BC film is stored under sterile conditions.

The method in which dried BC is exposed to UV-C radiation using lamps with a total power of 12W and maximum emission at 254 nm, for a period of 30 minutes, while maintaining sterility.
The method in which dried BC film is soaked in sterile distilled water at room temperature for up to 120 minutes and stored under refrigerated conditions while remaining sterile.
The method where, after exposure, it is soaked in sterile distilled water at room temperature for up to 120 minutes and stored under refrigerated conditions while remaining sterile.
The object of the invention is also an implantation kit, which contains the element specified above for use as a heart valve in cardio surgery.

## Description of the figures:

Fig.1 shows a view of an element for manufacturing a heart valve 1.
Fig.1a shows a plan view of the element for manufacturing the heart valve 1.
Fig.2 shows a plan view of a first embodiment of the element for manufacturing the heart valve 1.
Fig.3 shows a plan view of a second embodiment of the element for manufacturing the valve with cusps.
Fig.4A shows a diagram of areas of the element marked on Fig.1, Fig.1a and/or Fig.2 with number II, which are "T" shaped overlaps, by connecting them with the wall of the tube.
Fig.4B shows a diagram of areas of the element marked on Fig.3 with number II, which are "T" shaped overlaps, by connecting them with the wall of the tube.
Fig.5a represents the tensile strength held in physiological saline solution.
Fig.5b represents the tensile strength fixed in glutaraldehyde.
Fig. 5c shows the tensile strength of: BC native sheets; composites: BC-polyvinyl alcohol PVA, BC-hyaluronic acid.
Fig.5d represents tensile strength: BC sheets modified: BC dry s, BC 9 days, BC 8 days.

**Fig.5e** represents the tensile strength: BC sheets modified: BC with added aminobac.

**Fig.6** shows the values of elastic modulus and strain in the breaking test measured in the peripheral and axial direction.

**Fig.7** shows the values of breaking energy measured in the axial and peripheral direction.

**Fig.8** presents hysteresis test before dynamic fatigue test.

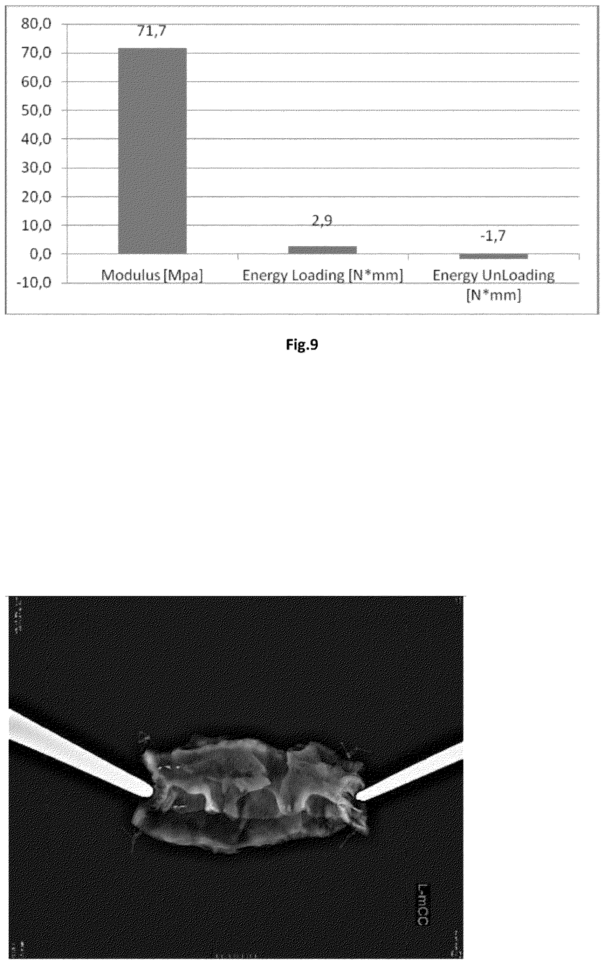

**Fig. 9** shows the hysteresis test after the dynamic fatigue test.

**Fig. 10** shows the X-ray of the valve implanted to sheep no. 1261.

**Fig. 11** shows the X-ray of the valve implanted to sheep no. 458.

**Fig. 12** shows the valve in histopathological examination.

**Fig. 13** shows the determination of calcium content in samples.

The invention is illustrated by the following examples, which do not limit the scope thereof.

## Example 1

Sterilized BC films were laid on a flat surface and dried to constant weight at room temperature not exceeding 25°C (convection drying, carried out in a dryer without forced circulation at 25°C. The drying agent was atmospheric air). The films were then packed and sterilized in an autoclave at 121°C for 20 minutes. Dried BC films were stored sterile.

## Example 2

BC films dried as in Example 1 were exposed to UV-C radiation with lamps with a total power of 12 W and maximum emission at 254 nm. Dried BC films were placed 5 cm away from the lamp and exposed for 30 min. BC films were stored sterile.

## Example 3

BC films prepared as in Example 1 were soaked in sterile distilled water at room temperature for up to 120 minutes and stored under refrigerated conditions, while sterile.

## Example 4

BC films prepared as in Example 2 were soaked in sterile distilled water at room temperature for up to 120 minutes and stored under refrigerated conditions, while sterile.

## Example 5

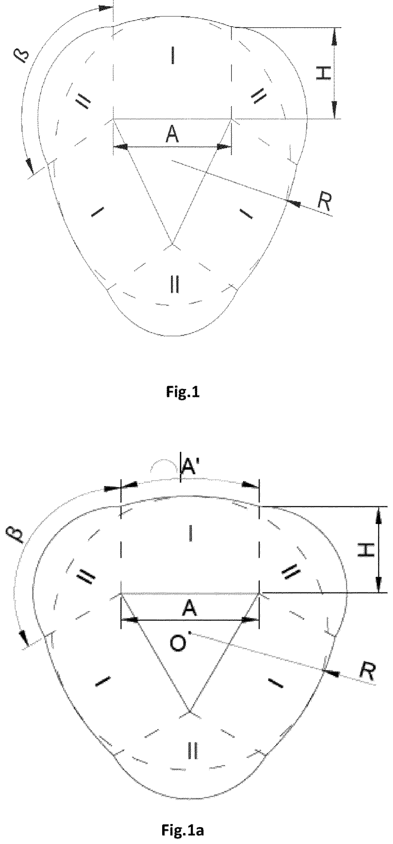

A heart valve component is made of a single sheet of biocompatible material, for example BC, as shown in Fig. 1a.

I denotes first areas of the element,
II denotes second areas of the element,
A denotes inner sides of the first areas I of the element,
$\beta$ denotes a central angle of the second areas II of the element which are in the form of circle slices,
H denotes the edges of second sides of the first areas I of the element,
O denotes a centre of symmetry of the central hole 1;
R denotes a radius of a circle with its centre coinciding with the centre of symmetry (O) of the central hole 1,
A' denotes the outer sides of the first areas (I) of the element.

The element for manufacturing a heart valve consisting of a flat sheet of BC with three axes of symmetry, having a centrally made hole 1 with three equal sides which form a figure with the symmetry of an equilateral triangle, such that

- edges of the hole 1 are inner sides A of first areas I of the element,
- the first areas I of the element are quadrilateral,
- second areas II of the element are placed between the first areas I of the element adjacent thereto,
- the second areas II of the element are circle slices having a central angle $\beta$ which is from $2\pi/3$ to $5\pi/6$ and radii equal to lengths of edges of second sides H of the first areas I of the element,
- the edges of the second sides H of the first areas I of the element are perpendicular to the inner sides A of the first areas of the element,
- the length of outer sides A' of the first areas I of the element is equal to the length of the first inner sides A of the first areas I of the element,
- the ratio of the length of radius I of a circle with its centre coinciding with a centre of symmetry O of the central hole 1 and tangent to the outer sides A' of the first areas I of the element, at mid-height length, to the length of the second sides H of the first areas I of the element is between $2\pi/9$ and $5\pi/6$,
- the ratio of the length of the first inner sides A of the first areas I of the element to the second sides H of the first areas I of the element is between $\pi/3$ and $2\pi/3$.

The edges of the central hole 1, which is an equilateral triangle, are the first sides A of the first areas I of the element. The first areas I of the element are similar in shape to a quadrilateral whose outer edge is convexly rounded. The arc of this rounding at its highest point is tangential to a circle having radius R equal to the length of the edge A, and of the centre coinciding with the centre of the central hole 1. Between the first areas I of the element there are immediately adjacent second areas II of the element II whose outer edges are convexly rounded.

The second H sides of the first areas I are perpendicular to the edge A of the central hole 1. The measure of the angle β between the second sides 2 is $2\pi/3$ and the relationship between the length of the edge A and the length of the second H side of the first area I is

$$H = \frac{2A}{\pi}\ .$$

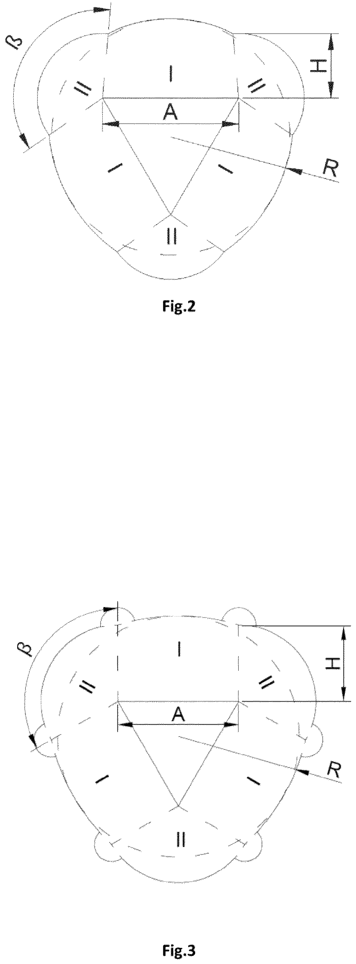

From the element created by a suitable, known anastomosis, for example stitching, a valve is formed such that the second areas of the second element bend outwards and form T-shaped fasteners in the tube in which the valve is to operate, as shown in Fig. 3 in a top view

$$R = A$$

$$\beta = \frac{2}{3}\pi$$

$$\frac{A}{H} = \frac{1}{2}\pi$$

$$\frac{R}{H} = \frac{A}{H}$$

| Parameter | | Example 5 |
|---|---|---|
| A | [mm] | 21.0 |
| R | [mm] | 21.0 |
| H | [mm] | 13.4 |
| β | [rad] | 2.09 |

The element for manufacturing a heart valve as shown in Fig.1a is made of a single flat sheet of BC with three axes of symmetry, having a centrally made hole 1 with three equal sides which form a figure with the symmetry of an equilateral triangle, such that

- edges of the hole 1 are inner sides A of first areas I of the element,
- the first areas I of the element are quadrilateral,
- second areas II of the element are placed between the first areas I of the element adjacent thereto,
- the second areas II of the element are circle slices having a central angle β which is from $2\pi/3$ to $5\pi/6$ and radii equal to the lengths of edges of second sides H of the first areas I of the element,

- the edges of the second sides H of the first areas I of the element are perpendicular to the inner sides A of the first areas I of the element,
- the length of outer sides A' of the first areas I of the element is equal to the length of the first inner sides A of the first areas I of the element,
- the ratio of the length of a radius R of a circle with its centre coinciding with the centre of symmetry O of the central hole 1 and tangent to the outer sides A' of the first areas I of the element, at mid-height length, to the length of the second sides H of the first areas I of the element is between $2\pi/9$ and $5\pi/6$,
- the ratio of the length of the first inner sides A of the first areas I of the element to the second sides H of the first areas I of the element is between $\pi/3$ and $2\pi/3$.

**Example 6**

The element for creating the heart valve as shown in Fig. 2 is manufactured as in Example 1, but for an angle of β of $2\pi/3$, the relationships between the radius of the circle R and the length of the edge A of the central hole 1 and the length of the other side H are:

$$H = \frac{3A}{2\pi}$$

$$R = \frac{7A}{8}$$

$$\beta = \frac{2}{3}\pi$$

$$\frac{A}{H} = \frac{2}{3}\pi$$

$$\frac{R}{H} = \frac{7\pi}{12}$$

From the element created by a suitable, known anastomosis, for example stitching, a valve is formed such that the second areas of element II are bent outwards and form T-shaped fasteners in the tube in which the valve is to operate, as shown in Fig. 3 in a top view.

| Parameter | | Example 6 |
|---|---|---|
| A | [mm] | 29.3 |
| R | [mm] | 34.3 |
| H | [mm] | 18.7 |

(continued)

| Parameter | | Example 6 |
|---|---|---|
| β | [rad] | 2.09 |

## Example 7

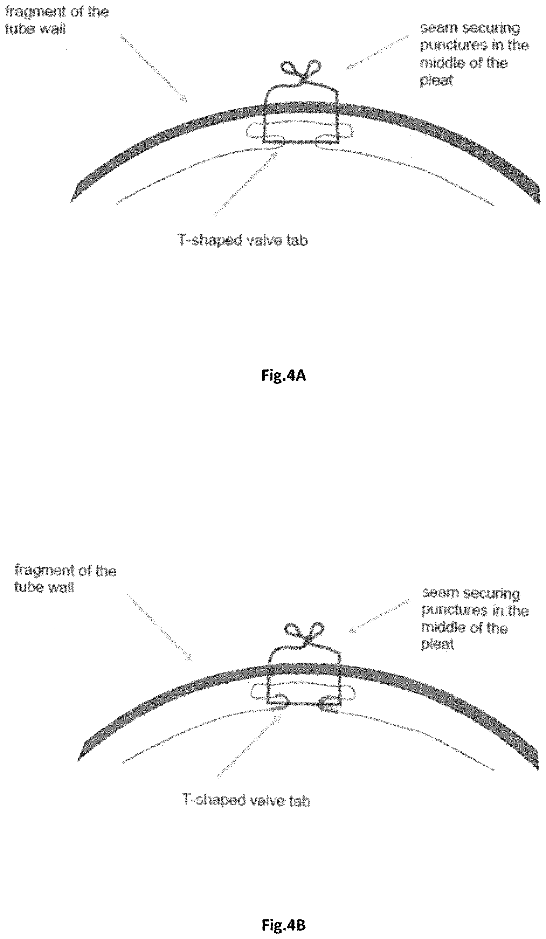

The element as in Example 5 was cut out from BC obtained as in Examples 1-4, additionally forming reinforcing cusps C. During the stitching process, the reinforcing cusps C are bent downwards, to the outer side of the valve, as schematically shown in Fig. 4B.

## Example 8

The suture method involves determining three points every 120 degrees, on the circumference of the circle in the tube in which the valve is to operate. This ring will be the place where the edges of the central opening of the element will be connected with the tube in which the valve will function. The tube may be made of artificial material or natural tissues.

The connection between the edges of the central opening and the ring may be made by continuous or interrupted surgical suture.

The areas of the element marked on Fig. 2 with number II constitute "T" shaped overlaps by connecting them with the wall of the tube according to the diagram in Fig. 3. The height of this connection is determined by H, counted from the points of stitching of the corners of the central hole upwards on the wall of the tube, evenly located above the points of the corners.

In this way the lower level of the connection between the valve inside the tube's light and the element remains completely tight - impermeable to liquid, and the stitching of "T" shaped overlaps from the top will cause the liquid flowing from the top to the bottom in the tube to produce a tight adherence of the areas of the element numbered I in Fig. 2. The liquid flowing inversely will cause the free opening of the areas I and thus the free flow of the liquid only in this direction.

## Example 9

Modifications of BC aiming at obtaining a material resistant to enzymes and selected pathogenic microorganisms and determining the influence of these modifications on other properties of the polymer.

Resistance of modified BC to in vitro degradation was investigated by determining the changes occurring in the polymer during incubation of samples at 37oC in a solution simulating physiological fluids in the absence and presence of *Aspergillus fumigatus.*

Modification of BC:

BC samples (measuring 2.5 x 2.5 cm) modified by drying at room temperature and soaking in water and by drying at room temperature, exposed to UV radiation and soaked in water were placed in sterile bottles of 100 mL filled with 62.5 mL of sterile SBF liquid. For the study of mechanical properties, modified BC strips (1.5 x 10 cm) were placed in 150 mL of the solution in sterile bags. A sufficient amount of A. fumigatus liquid fungus culture was added to a part of the samples immersed in SBF solution so that the initial number was about 103 cfu per 1 mL of liquid. Degradation changes of BC samples were examined after 3, 7, 14, 30 days from the beginning of incubation.

Determination of BC biodegradability degree:

1. determination of BC wet mass changes
2. examination of mechanical properties of BC
3. examination of thermal and structural properties of BC
4. X-ray diffraction analysis (XRD)
5. microscopic analysis using SEM technique

The results of the experiment were developed using the statistical program SigmaPlot 11.0 (SYSTAT Software, Germany), with ANOVA one-way analysis of variance for significance level $p < 0.05$.

The wet mass of BC samples modified by drying at room temperature and soaking in water and then stored in sterile SBF fluid for 3, 7 and 14 days increased twice, and after 30 days of incubation no statistically significant differences were observed. In the case of samples incubated in the presence of *A. fumigatus,* greater changes in wet mass were observed than in the case of BC samples stored under sterile conditions. After only 3 days of incubation, wet mass increased about 2.5-fold, and after 7 days - 4-fold.

Different results were obtained during storage of BC samples modified by drying at room temperature, UV radiation and soaking in water. In this case, incubation in sterile SBF fluid resulted in a decrease in the content of BC wet mass by approx. 60% (Fig. 3), and incubation in SBF fluid in the presence of *A. fumigatus* resulted in a decrease in this content by approx. 50% only after 3 days of incubation.

The stress at a break ($\sigma$) of non-incubated BC samples modified by drying at room temperature and soaking in water was about 112 MPa and the relative elongation at break ($\varepsilon$) about 13%. In the case of samples modified by drying at room temperature, UV radiation and soaking in water, $\sigma$ of the non-incubated membranes was about 160 MPa and $\varepsilon$ about 9%.

Storage both in sterile SBF fluid and in the presence of *A. fumigatus* deteriorated the mechanical strength of BC membranes modified by drying at room temperature and soaking in water. Already after 3 days of incubation in sterile SBF fluid a decrease in $\sigma$ by about 60% was observed, after 7 days by about 20%, and after 14 and 30 days by about 50%. Incubation in the presence of *A. fumigatus* for the period of 3 days caused a similar

decrease in $\sigma$ as in the case of fungus-free incubation by about 60%, but after 14 and 30 days $\sigma$ decreased significantly more, by 70 and 90% respectively. $\varepsilon$ of the samples did not change during the whole incubation period in sterile SBF fluid, whereas in the presence of *A. fumigatus* it decreased by about 30% after 14 days of incubation and by about 70% after 30 days. The values of $\sigma$ and $\varepsilon$ of BC samples dried at room temperature, exposed to UV radiation and soaked in water and then incubated in sterile SBF fluid did not change in comparison to non-incubated samples, except for $\varepsilon$ samples stored for 14 days, the value of which increased by about 20%. On the other hand, BC samples incubated in SBF fluid in the presence of A. fumigatus showed about 20 and 60% lower $\sigma$ value after 7 and 30 days of incubation respectively, in comparison to radiated and non-incubated samples. $\varepsilon$ of these samples did not change, except for samples incubated for 14 days, which increased by about 30%.

When analysing the diffractograms of all modified BC samples, 3 characteristic diffraction bands were observed at the reflective angle of $2\theta$ 14.46°, 16.7° and 22.62°. The incubation of the samples under the conditions simulating body fluids did not affect the position of diffraction lines but caused a change in the intensity of diffraction bands at the angles of reflection $2\theta$ 14.46° and 22.62°. Additionally, it was noted that in the case of the diffractogram of BC dried at room temperature and soaked in water and then stored for 3 days in both sterile SBF and liquid SBF in the presence of A. fumigatus, the intensity of the diffraction line is increased at an angle of $2\theta$ 16.7°. The crystallinity degree (Cr.I.) of all modified BC samples calculated on the basis of the formula of Segal et al. (1959) was about 90%.

Changes in BC crystallinity caused by biodegradation of samples modified by drying at room temperature and soaking in water, drying at room temperature, UV radiation and soaking in water were compared. The crystallinity degree of all tested samples differed only slightly. The exception was BC dried at room temperature and soaked in water and then kept for 3 days in the presence of fungus. In this case, a decrease in Cr.I. by about 5% was observed in comparison with the non-incubated sample.

The decomposition temperature of BC that is not incubated, dried at room temperature, UV-radiated and soaked in water was approximately 6°C lower than that of BC that is dried at room temperature and soaked in water. A lower decomposition temperature indicates lower thermal stability of the material, probably due to greater susceptibility to biodegradation of the material. SEM images of the surface of all BC samples modified and then incubated in sterile SBF fluid did not show any differences compared to the surface of non-incubated samples. Hardly visible, thin fibres can be observed on the sample surface. A more compact structure of BC samples incubated in sterile SBF fluid may be the reason for its increased mechanical strength in comparison with the samples incubated in SBF in the presence of *A. fumigatus.*

Obtaining BC with greater resistance to biodegradation characteristic for bio-prostheses of blood vessels and aortic valves.

## Example 10

Mechanical testing of BC and BC-based composite materials.

The conducted research allowed to choose BC with no worse tensile strength than natural tissues of the swine cardiovascular system. In order to achieve this, BC sheets of 10 x 1.5 cm in size were subjected to a tensile test. The reference material were natural tissues: aorta, aortic valve and pericardial sac fragments. Natural tissues were properly prepared and supplied by the Medical University of Gdansk. The tissues were divided into two groups. The first group consisted of tissues stored in physiological saline solution, from the moment the samples were delivered to the moment the tensile test was performed. However, the second group consisted of tissues additionally washed with 0.5% glutaraldehyde for 10 minutes prior to the tensile test.

The material is: Native BC, Modified BC and Composite BC-Polyvinyl alcohol, BC-hyaluronic acid.

The tensile test was carried out on an INSTRON model 1112 testing machine with a single-axis tensile velocity of 5 mm/s. The distance between the jaws for BC stretching was 50 mm.

Studies show that thermally modified BC, i.e. dried "s" and then soaked, has the best tensile strength of all bio-nanocellulose materials and has a strength of 22 MPa. Native (homogeneous) BC and BC-based composite materials have a tensile strength of about 5 MPa, which is lower than that of natural materials. The value of approx. 22 MPa corresponding to thermal modified BC allows for further strength testing (tear test, fatigue test) on a selected BC material, which may be a potential material used in cardio surgery and vascular surgery.

The susceptibility of BC to *in vitro* degradation in simulated body fluid (SBF at 37°C) was determined. Various methods of monitoring degradation changes were applied, e.g. determination of dry and wet mass changes, determination of biomaterial hydrolysis products using liquid chromatography and thermal stability of BC. The surface of biomaterial was evaluated by scanning electron microscopy (SEM), and the development of microorganisms deliberately introduced into the environment in which BC was incubated was also monitored.

During the storage of BC samples in sterile SBF fluid and PSB buffer no changes in dry matter were found for the whole 6-month storage period. Furthermore, in the presence of S. *aureus* bacteria and C. *albicans* yeast, the dry matter of BC samples remained at a similar level. Significant decrease in BC dry matter was observed only in the samples incubated for 6 months in the presence of A. *fumigatus* fungus - BC dry matter decreased by 41%. In the case of wet mass, it was found that it significantly increased after the second month of storage, regardless of the conditions (PBS buffer, SBF liquid in the presence and absence of microorganisms).

Moreover, in the presence of *S. aureus* bacteria and C. *albicans* yeast, the dry matter of BC samples remained at a similar level. Significant decrease in BC dry matter was observed only in the samples incubated for 6 months in the presence of *A. fumigatus* fungus - BC dry matter decreased by 41%. In the case of wet mass, it was found that it significantly increased after the second month of storage, regardless of the conditions used (PBS buffer, SBF liquid in the presence and absence of microorganisms).

It was also shown that during the storage of BC samples, the growth of all tested microorganisms occurred. After 1 month, the number of S. *aureus, C. albicans* and *A. fumigatus* cells increased from about $10^3$ to $10^5$ cfu/cm$^3$ and remained at the same level for up to 6 months, which indicates that they were in the stationary phase of growth. The growth of microorganisms and wet BC mass indicates that degradation changes occur in this material, although its dry mass does not change significantly (except for the samples containing *A. fumigatus* fungi). It was also found that during the storage of samples on the BC surface only A. *fumigatus* fungi formed a biofilm on the surface. The concentration of saccharides, which are the products of BC hydrolysis, in the post-incubation fluids was so low that they could not be detected by thin layer chromatography (TLC). After a 20-fold concentration of these fluids, both from samples with and without *S. aureus* bacteria and *C. albicans* yeasts, no BC hydrolysis products were found. However, they were present (though in small amounts) in a concentrated post-incubation fluid after only one month of BC treatment with *A. fumigatus* fungi. When analysing the obtained results, it can be stated, however, that the investigation of the presence of cellulose hydrolysis products in post-incubation fluids is not a good method to determine the degree of polymer biodegradation, because microorganisms can metabolize simple sugars, disaccharides and oligosaccharides produced in this process.

The storage of samples in PBS buffer and sterile SBF fluid for the period of 5 and 6 months resulted in a decrease in BC decomposition temperature by about 10°C, which indicates degradation processes and reduction of water adsorbed on its surface by half on average. Similar changes were observed in the samples stored in the presence of *S. aureus* bacteria and *C. albicans* yeasts. However, the samples stored in the presence of *A. fumigatus* fungus for 6 months had thermal stability decreased by approx. 20°C. The analysis of thermograms of the samples incubated in SBF fluid for 6 months in the presence and absence of microorganisms also showed an additional effect indicating the loss of chemically combined water.

Microscopic observations showed that the morphology of BC film surface after incubation in simulation fluids for the period of 1-6 months did not change significantly, it only became more porous.

## Example 11

The description of the tests that have been performed to assess the properties of BC.

The aim of the task was to assess the biomechanical properties of BC material *in vitro.*

*Tear test*

For each test, single-axis tear tests were performed using Tytron 250 Microforce Testing System (MTS) with a 250 N force transducer. Percentage strain was measured using a video extensometer (Messphysik). For a proper strain analysis, appropriate markers were used to determine the L0 and L1 parameters of the specimen during breaking test. Before each test, the extensometer was calibrated with the use of standards provided by the manufacturer.

For proper measurement, efforts were taken to maintain the width to length ratio of the specimen 1:10. Each time prior to the test, the thickness of the specimen was measured, which was an input to automatically obtain the cross-sectional area value and thus automatically recalculate the elasticity. In the case of tensile tests, the sample was preloaded with 0.5 N and the breaking test started from this value each time the test started.

The elasticity, percentage of real strain and breaking energy were measured. Taking into account the anisotropic character of BC material, the tests were performed in two conventional peripheral and axial directions. Prior to the tests, the tested material was weighed each time; it resulted from the fact that BC has strong hydrophilic properties, which allowed to obtain information on the extent to which the biomechanical parameters could be influenced by the degree of hydration of the sample.

*2. Viscoelastic properties test*

Viscoelastic properties of BC samples were also tested by hysteresis. Prior to the test, the specimens were preloaded to 1 N, then stretched to 4% strain and maintained for 60 seconds and then returned to their initial values. The hysteresis value was measured as the difference between the input and output energy.

*3. Dynamic fatigue test*

Dynamic fatigue test was performed at 370 °C in an environmental chamber filled with DMEM/F12 medium supplemented with 10% serum and antibiotics. Similarly to the tensile tests, BC samples were weighed before and after the completion of the test. The test was performed at 5 mm amplitude at 3 Hz at 250,000 cycles. At the end of the fatigue test, the hysteresis of the specimen was determined each time.

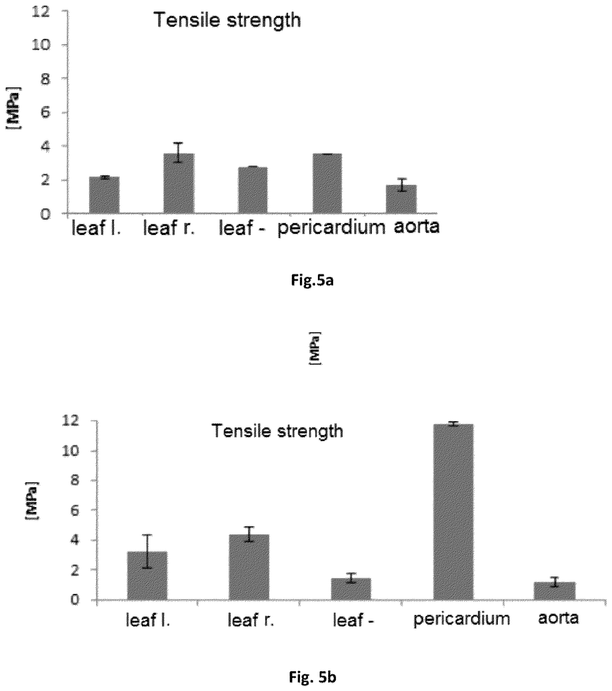

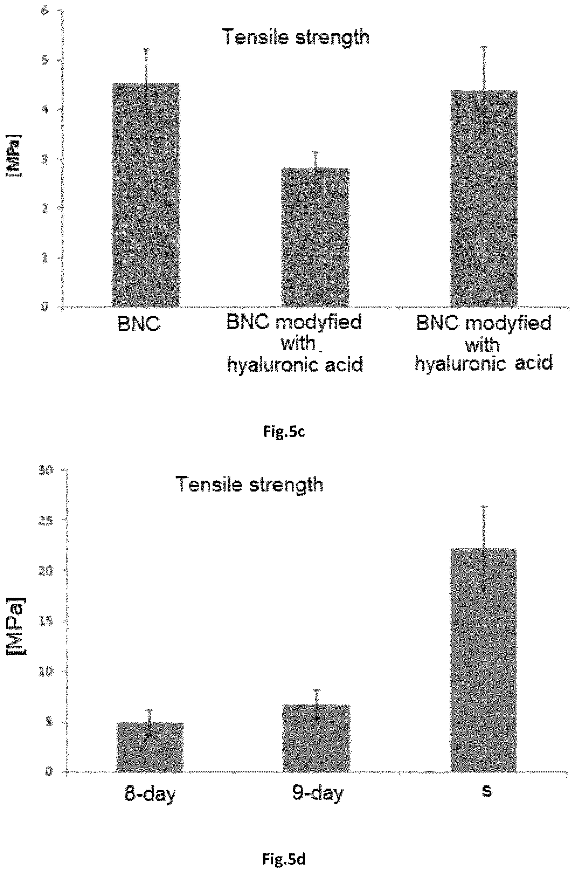

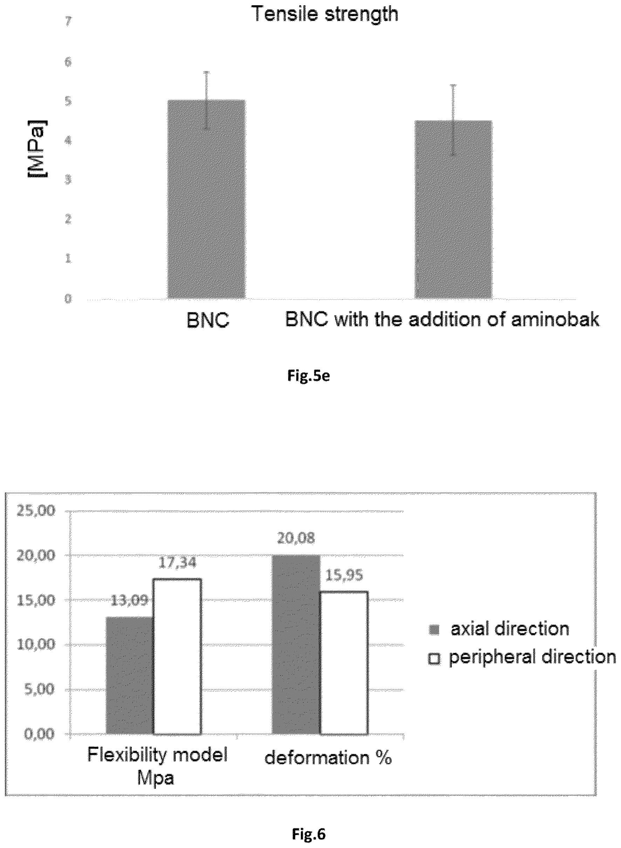

The elasticity, strain and breaking energy differed depending on the direction of the specimen arrangement (Fig. 5,6).

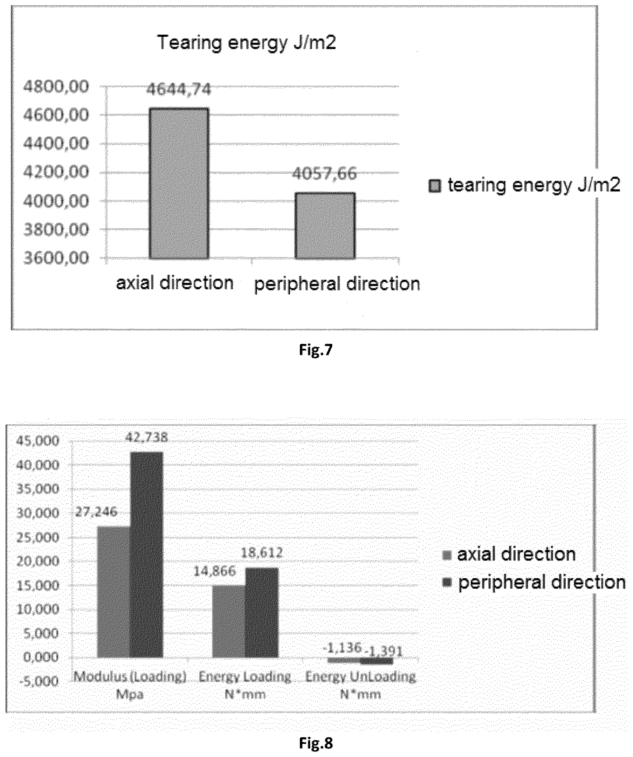

Hysteresis tests indicate that BC has poor viscoelastic properties. Due to the anisotropic character of the BC samples, the stiffness of the material was observed in a dynamic fatigue test (Fig. 7, 8).

Biomechanical properties indicate that the studied BC material has much higher stiffness in relation to human and swine tissues (pulmonary and aortic valves). The BC material has high hydrophilicity.

Features of the obtained valve:

For the material which is flat, flaccid and of the thickness between 300 and 600 micm as well as impenetrable for the morphotic components of blood:

**1.tensile strength exceeding 5 MPa**

native 5 MPa, modified 22 MPa
Studies show that thermally modified BC, i.e. dried "s" and then soaked, has the best tensile strength of all bio-nanocellulose materials and has a strength of 22 MPa. Native (homogeneous) BC and BC-based composite materials have a tensile strength of about 5 MPa, which is lower than that of natural materials. The value of about 22 MPa corresponding to the thermal modified BC allows to continue the strength tests (tear test, fatigue tests) on the selected BC material, which may be a potential material used in cardio surgery and vascular surgery.

**2. fatigue resistance with the following parameters:**

force 45 N
vibration amplitude A=5 mm
frequency f=3 Hz
time 24 hours

3. **biostability** (after 6 months of incubation in sterile PBS buffer and sterile SBF fluid)
Storage of samples in sterile PBS buffer and sterile SBF fluid for the period of 5 and 6 months resulted in a decrease in BC decomposition temperature by about 10°C, which indicates degradation processes and the reduction of water adsorbed on its surface by half on average. Similar changes were observed in the samples stored in the presence of *S. aureus* bacteria and *C. albicans* yeasts, whereas the samples stored in the presence of *A. fumigatus* fungus for 6 months had their thermal stability decreased by approx. 20°C. The analysis of thermograms of samples incubated in SBF fluid for 6 months in the presence and absence of microorganisms also showed an additional effect indicating the loss of chemically combined water.

Microscopic observations showed that the morphology of BC membranes surface after incubation in simulation fluids for the period of 1-6 months does not change significantly, it only becomes more porous.

4. **low haemolysis index**
5. **low thrombogenicity**

The study was performed on fresh heparinized swine blood obtained during traditional slaughter of animals in a slaughterhouse. A total of 13 experimental sessions were conducted - 7 without BC (control) and 6 with the use of BC (experimental). At half-hour intervals, activated coagulation time (ACT), total haemoglobin (Hb), free haemoglobin (fHb), erythrocyte count (RBC) and haematocrit (HTC) were examined.

ACT monitoring was an element of the methodology applied, i.e. the Schima test. This parameter, which is an indicator of the tendency of blood to coagulate, determines the moment of termination of the experiment and is not useful in itself for the assessment of the degree of haemolysis or thrombogenicity of the tested material. HCT, RBC and Hb changed slightly during the study and did not exceed the norms given in the literature for swine. fHb in plasma is closely correlated with the haemolysis process in the circulating medium which was studied. During the studies (both control and with the use of BC) it was subject to a gradual, systematic increase.

The comparison of fHb growth dynamics during control and experimental studies allowed to make a conclusion concerning the influence of BC on haemolysis. Since it is affected by both blood parameters (haematocrit, initial concentration of free haemoglobin) as well as pumping time, the obtained data required standardization. For this purpose, IH index (Free Haemoglobin Index) was used to determine an increase in plasma free haemoglobin content in mg/l of pumped blood. This is particularly important due to differences in the duration of individual trials. IH was calculated with the formula:

$$IH = \frac{\Delta Hg * (100 - HTC)}{Q * T}$$

$\Delta$Hg [mg/l] - difference between the concentration of free haemoglobin in the plasma between the first and the last measurement
HTC [%] - Haematocrit
Q [l/min] - Flow
T [min] - Flow time

The mean (M±SD) haemoglobin index for control samples was equal to: IH = 14.71±9.42, and for experimental IH = 12.87±3.52, while the mean (M±SD) circulating medium flow time (M±SD) for control samples T = 325.57±75.08 min., and for experimental T = 330.00±60.75 min. At the same time the differences between these mean pairs (control/experiment) turned out to be statistically insignificant (p>0.05).

It is worth noting that despite a slightly longer time of pumping blood, a contact with BC resulted in a lower haemolysis index than for control samples.

Thrombogenicity testing according to the Schima protocol is limited to the assessment of the extent to which the surface of the tested material is covered by thrombi. Macroscopic evaluation of BC fragments after the completed experiments in most cases showed the presence of relatively small number of red thrombi, especially on the surface in direct contact with flowing blood. The number of observed thrombi were even lower on the "opposite" surfaces of scraps loosely adjacent to the walls of flow channels of the equipment.

The Schima BC test does not show a significant haemolytic activity and its thrombogenicity seems to be insignificant.

6. **low adhesiveness**

BC is characterized by low adhesiveness. Lack of proper adhesion of cells affects the generation of necrotic processes. Surface modification of BC with the use of natural proteins of intracellular matrix significantly improves the adhesiveness of cells and growth characteristic, what may have a significance in the case of clinical application of BC.

**Example 12**

Implantation of bio-nanocellulose valve performed in sheep

Sheep numbers:

    1261

    4466

    4584

All implants of the valves were manufactured according to the protocol. During the first week after the implantation of the valves (first post-operative week), no adverse events were reported. All three sheep kept their normal appearance, general health and were not feverish. The recovery occurred in so-called normal status.

Then the sheep underwent a planned indirect ECG (within the period of 3 months). The sheep maintained a very good clinical condition. All echocardiograms show that the valves are in a good clinical condition. 6 months after the operation, the sheep underwent echocardiography again with further maintenance of good health condition. The echocardiography was performed inside the thorax cavity and the explant. Good elasticity and valve cusp movement were stated. Distinctively blocked or calcified valve cusp were not noted.

In the results of the sheep's blood tests, no significant abnormalities were detected. No signs of blood cells damage, haemolysis or infection.

Summing up:

Lack of significant insufficiency. Good movement of the cusps of the valve, good elasticity.

Micro CT scan

Scans reveal new structural defects in the valve cusps. There is a presence of minimum calcification, mainly in sheep no. 4466. The lowest calcification is seen in sheep no. 1261. Due to the hypertrophy of new tissues, there is a slight thickening of the wall and cusps both from the side of the tunica intima (inner) and also from the tunica externa (outermost).

The median calcium content is only 1.88 $\mu$g / mg tissue, between 0.48 and 9.63 (IQR 0.56-4.51). The above graph shows the mean +/- SE and 95% CI.

Summary:

The valve functions properly in the pulmonary position, more than 6 months after the pulmonary valve replacement surgery.

No surgical failure was noted. No damage to the material occurred. Lack of evidence of blood or platelet damage.

Low gradients and minor insufficiency were observed.

Only slight, temporary external calcifications connected with the suture were observed.

Good general clinical condition of animals.

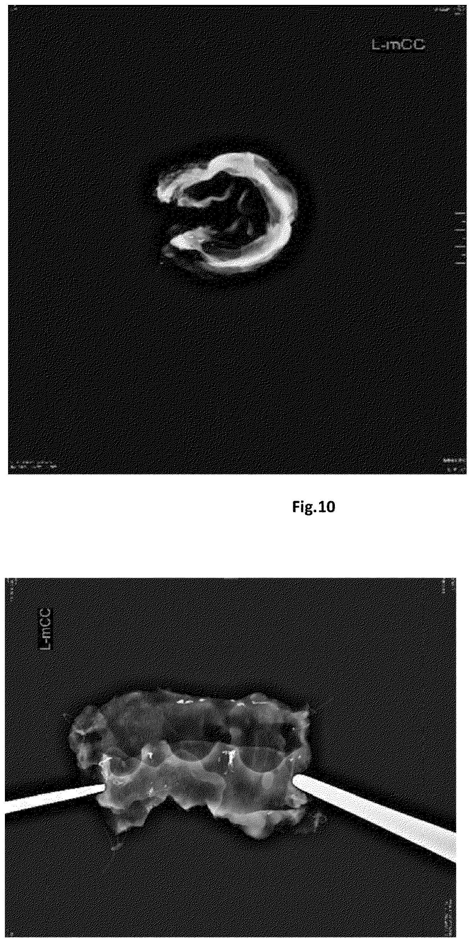

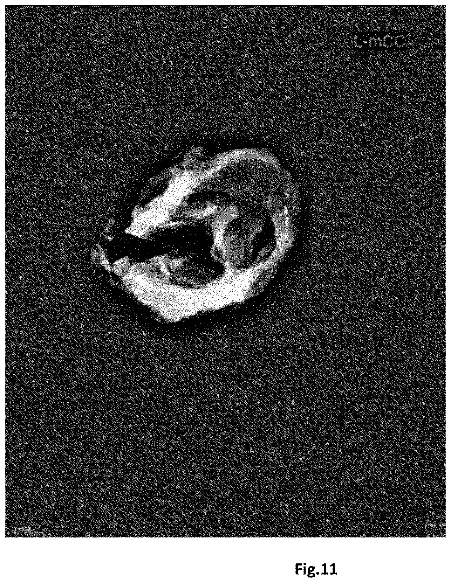

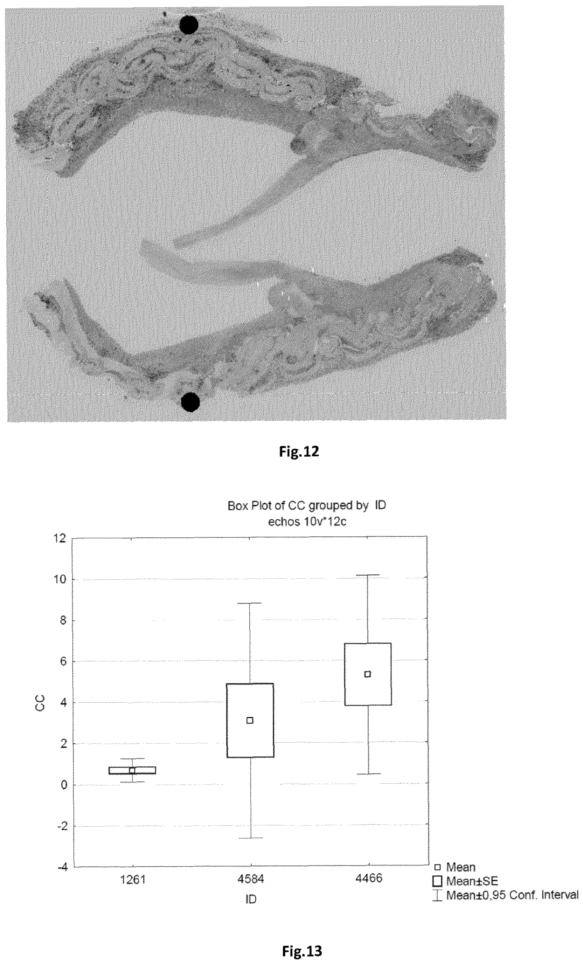

Histological result shows the lack of damage to the material, lack of calcification in the materials, only minor external calcification towards the matrix (Figs. 10-13).

**Claims**

1. An element for manufacturing a heart valve comprising of a flat sheet of BC with three axes of symmetry, having a centrally made hole with three equal sides which form a figure with the symmetry of an equilateral triangle, **characterised in that**

    - edges of the hole (1) are inner sides (A) of first areas (I) of the element,
    - the first areas (I) of the element are quadrilateral,
    - second areas (II) of the element are placed between the first areas (I) of the element adjacent thereto,
    - the second areas (II) of the element are circle slices having a central angle ($\beta$) which is from

$2\pi/3$ to $5\pi/6$ and radii equal to the lengths of edges of second sides (H) of the first areas (I) of the element,

- the edges of the second sides (H) of the first areas (I) of the element are perpendicular to the inner sides (A) of the first areas (I) of the element,
- the length of outer sides (A') of the first areas (I) of the element is equal to the length of the first inner sides (A) of the first areas (I) of the element,
- the ratio of the length of a radius (R) of a circle with its centre coinciding with a centre of symmetry (O) of the central hole (1) and tangent to the outer sides (A') of the first areas (I) of the element, at mid-height length, to the length of the second sides (H) of the first areas (I) of the element is between $2\pi/9$ and $5\pi/6$,
- the ratio of the length of the first inner sides (A) of the first areas (I) of the element to the second sides (H) of the first areas (I) of the element is between $\pi/3$ and $2\pi/3$.

2. The element according to claim 1 wherein the element is a bi-coherent figure.

3. The element according to claim 1 wherein the axes of symmetry of the element and the hole (1) overlap.

4. The element according to any claim 1 to 6 wherein the first areas (I) of the element meet the second areas (II) of the element and wherein the element has strengthening knobs (2) on an outer side of the element which are preferably semi-circular.

5. A method for producing the modified BC film of claim 1 by using a *G. xylinus* strain wherein sterilized BC film is laid on a flat surface and dried to constant weight at room temperature not exceeding 25°C, then the film is sterilized at 121°C for 20 minutes and the resulting BC film is stored under sterile conditions.

6. The method according to claim 5 wherein the dried BC film is exposed to UV-C radiation using lamps with a total power of 12W and maximum emission at 254 nm, for a period of 30 minutes, while maintaining sterility.

7. The method according to claim 5 wherein the dried BC film is soaked in sterile distilled water at room temperature for up to 120 minutes and the BC film is stored under refrigerated conditions while remaining sterile.

8. The method according to claim 6 wherein the BC film after UV-C exposure is soaked in sterile distilled water at room temperature for up to 120 minutes and stored under refrigerated conditions while remaining sterile.

9. A kit comprising the element as defined in claim 1 for use as a heart valve in cardio surgery.

**Patentansprüche**

1. Element zur Herstellung einer Herzklappe, umfassend ein flaches Blatt aus BC mit drei Symmetrieachsen, das ein mittig ausgeführtes Loch mit drei gleichen Seiten aufweist, die eine Figur mit der Symmetrie eines gleichseitigen Dreiecks bilden, **dadurch gekennzeichnet, dass**

- Ränder des Lochs (1) Innenseiten (A) erster Bereiche (I) des Elements sind,
- die ersten Bereiche (I) des Elements viereckig sind,
- zweite Bereiche (II) des Elements zwischen den daran angrenzenden ersten Bereichen (I) des Elements angeordnet sind,
- die zweiten Bereiche (II) des Elements Kreisausschnitte mit einem Zentriwinkel (β) von $2\pi/3$ bis $5\pi/6$ und Radien gleich den Längen der Ränder zweiter Seiten (H) der ersten Bereiche (I) des Elements sind,
- die Ränder der zweiten Seiten (H) der ersten Bereiche (I) des Elements senkrecht zu den Innenseiten (A) der ersten Bereiche (I) des Elements sind,
- die Länge der Außenseiten (A') der ersten Bereiche (I) des Elements gleich der Länge der ersten Innenseiten (A) der ersten Bereiche (I) des Elements ist,
- das Verhältnis der Länge eines Radius (R) eines Kreises, dessen Mittelpunkt mit einem Symmetriezentrum (O) des zentralen Lochs (1) zusammenfällt und der tangential zu den Außenseiten (A') der ersten Bereiche (I) des Elements ist, auf halber Höhe der Länge, zur Länge der zweiten Seiten (H) der ersten Bereiche (I) des Elements zwischen $2\pi/9$ und $5\pi/6$ liegt,
- das Verhältnis der Länge der ersten Innenseiten (A) der ersten Bereiche (I) des Elements zu den zweiten Seiten (H) der ersten Bereiche (I) des Elements zwischen $\pi/3$ und $2\pi/3$ liegt.

2. Element nach Anspruch 1, wobei das Element eine bi-kohärente Figur ist.

3. Element nach Anspruch 1, wobei sich die Symmetrieachsen des Elements und des Lochs (1) überlappen.

4. Element nach einem der Ansprüche 1 bis 6, wobei die ersten Bereiche (I) des Elements die zweiten Bereiche (II) des Elements treffen und wobei das Element Verstärkungsknöpfe (2) an einer Außenseite des Elements aufweist, die vorzugsweise halbkreisförmig sind.

**5.** Verfahren zur Herstellung der modifizierten BC-Folie nach Anspruch 1 unter Verwendung eines *G. xylinus*-Stammes, wobei sterilisierte BC-Folie auf eine flache Oberfläche gelegt und bei Raumtemperatur, die 25°C nicht übersteigt, bis zu einem konstanten Gewicht getrocknet wird, dann die Folie bei 121°C für 20 Minuten sterilisiert wird und die resultierende BC-Folie unter sterilen Bedingungen gelagert wird.

**6.** Verfahren nach Anspruch 5, wobei die getrocknete BC-Folie unter Verwendung von Lampen mit einer Gesamtleistung von 12 W und einer maximalen Emission bei 254 nm 30 Minuten lang unter Beibehaltung der Sterilität UV-C-Strahlung ausgesetzt wird.

**7.** Verfahren nach Anspruch 5, wobei die getrocknete BC-Folie in sterilem destilliertem Wasser bei Raumtemperatur bis zu 120 Minuten lang eingeweicht wird und die BC-Folie unter Kühlbedingungen unter Beibehaltung der Sterilität gelagert wird.

**8.** Verfahren nach Anspruch 6, wobei die BC-Folie nach der UV-C-Exposition bis zu 120 Minuten in sterilem destilliertem Wasser bei Raumtemperatur eingeweicht und unter Kühlbedingungen unter Beibehaltung der Sterilität gelagert wird.

**9.** Kit umfassend das in Anspruch 1 definierte Element zur Verwendung als Herzklappe in der Kardiochirurgie.

**Revendications**

**1.** Élément pour fabriquer une valve cardiaque comprenant une feuille plate de BC (cellulose bactérienne) présentant trois axes de symétrie, présentant un creux situé centralement ayant trois côtés égaux qui forment un figure présent une symétrie d'un triangle équilatéral, **caractérisé en ce que** :

- bords du creux (1) sont des côtés intérieurs (A) des zones premières (I) d'élément,
- zones premières (I) d'élément sont quadrilatérales,
- zones secondes (II) d'élément sont situées entre les zones premières (I) d'élément adjacentes à celles-ci,
- zones secondes (II) d'élément sont des secteurs circulaires ayant un angle au centre qui est de 2π/3 à 5π/6 et rayons étant égaux aux longueurs des bords des côtés secondes (H) des zones premières (I) d'élément,

- les bords des côtés secondes (H) des zones premières (I) d'élément sont perpendiculaires aux côtés intérieurs (A) des zones premières (I) d'élément,
- la longueur des côtés extérieurs (A') des zones premières (I) d'élément est égale à la longueur des côtés intérieurs premiers (A) des zones premières (I) d'élément,
- le rapport de la longueur d'un rayon (R) d'un cercle ayant sa centre coïncidant avec un centre de symétrie (O) du creux central (1) et tangente aux côtés extérieurs (A') des zones premières (I) d'élément, à mi-longueur, à la longueur des côtés secondes (H) des zones premières (I) d'élément est compris entre 2π/9 et 5π/6,
- le rapport de la longueur des côtés intérieurs premiers (A) des zones premières (I) d'élément aux côtés secondes (H) des zones premières (I) d'élément est compris entre π/3 et 2π/3.

**2.** Élément selon la revendication 1, l'élément étant une figure bicohérente.

**3.** Élément selon la revendication 1, les axes de symétrie d'élément et le creux (1) se recouvrant.

**4.** Élément selon l'une quelconque des revendications 1 à 6, dans lequel les zones premières (I) d'élément se rejoignent avec les zones secondes (II) d'élément et l'élément présentant des boutons de renforcement (2) sur un côté extérieure d'élément qui sont de préférence semi-circulaires.

**5.** Procédé pour fabriquer une feuille de BC modifié de revendication 1 en utilisant une souche bactérienne de *G. xylinus,* une feuille de BC stérilisée étant placée sur une surface plate et séchée jusqu'à ce qu'elles atteignent un poids constant à température ambiante jusqu'à 25°C, ensuite la feuille est stérilisée à 121°C pour 20 minutes et la feuille de BC résultant est conservée en milieu stérile.

**6.** Procédé selon la revendication 5, la feuille de BC séchée étant exposée aux rayons UV-C en utilisant des lampes présentant une puissance de 12W et un spectre d'émission maximale de 254 nm, pour une durée de 30 minutes tout en maintenant sa stérilité.

**7.** Procédé selon la revendication 5, la feuille de BC séchée étant mouillée dans l'eau distillée stérile à température ambiante pour une durée de temps allant jusqu'à 120 minutes, et la feuille de BC étant conservée dans des conditions réfrigérées tout en restant stérile.

**8.** Procédé selon la revendication 6, la feuille de BC étant mouillé dans l'eau distillée stérile à température ambiante pour une durée de temps allant jusqu'à 120 minutes après sa exposition aux rayons UV-C, et étant conservée dans des conditions réfrigérées tout en restant stérile.

9. Kit comprenant un élément tel que défini dans la revendication 1 pour utiliser en tant que une valve cardiaque en chirurgie cardiaque.

Fig.1

Fig.1a

**Fig.2**

**Fig.3**

fragment of the
tube wall

seam securing
punctures in the
middle of the
pleat

T-shaped valve tab

**Fig.4A**

fragment of the
tube wall

seam securing
punctures in the
middle of the
pleat

T-shaped valve tab

**Fig.4B**

Fig.5a

Fig. 5b

**Fig.5c**

**Fig.5d**

Fig.5e

Fig.6

**Fig.7**

**Fig.8**

**Fig.9**

**Fig.10**

Fig.11

**Fig.12**

Box Plot of CC grouped by ID
echos 10v*12c

□ Mean
□ Mean±SE
⊥ Mean±0,95 Conf. Interval

**Fig.13**

**REFERENCES CITED IN THE DESCRIPTION**

**Patent documents cited in the description**

- PL 180925 B
- EP 1083845 B
- PL 229562 B
- PL 190961 B1
- EP 346507 A
- PL 214844 B1
- US 20090220560 A
- PL 216702 B1
- WO 2016083352 A
- EP 2015077464 W
- WO 2013119912 A
- US 2013025287 W
- US 20090222085 A1
- US 6328763 B1